# EUROPEAN PATENT SPECIFICATION

(11) **EP 4 069 687 B1**
(45) Date of publication and mention of the grant of the patent: **31.01.2024**
(21) Application number: 20820820.7
(22) Date of filing: 04.12.2020
(51) Int. Cl.: C07D 403/04, C07D 405/14, C07D 487/04

(54) **PROCESS AND INTERMEDIATES FOR THE PRODUCTION OF FORMULA (I)**
VERFAHREN UND ZWISCHENPRODUKTE FÜR DIE PRODUKTION VON FORMEL (I)
PROCÉDÉ ET INTERMÉDIAIRES POUR LA PRODUCTION DE FORMULE (I)

(30) Priority: 05.12.2019 US 201962943851 P
(43) Date of publication of application: 12.10.2022
(73) Proprietor: Astrazeneca AB, 151 85 Södertälje (SE)
(72) Inventor: DUBIEZ, Jerome, Macclesfield, SK10 2NA (GB); TURNER, Andrew, Macclesfield, SK10 2NA (GB); CHUBB, Richard, Sunderland SR5 2TQ (GB)
(74) Representative: Simmons & Simmons LLP (Munich)
(86) International application number: PCT/EP2020/084580
(87) International publication number: WO 2021/110893

(56) References cited:
- WO-A1-2017/080979

## Description

The present specification relates to a process and intermediates for the production of the compound of Formula **(I):** 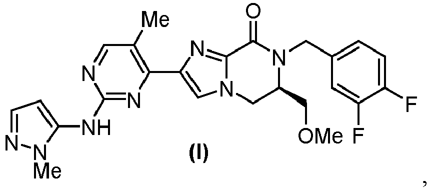 or a pharmaceutically acceptable salt or co-crystal thereof.

International patent application WO2017/080979 A1 describes the compound of Formula **(I)** (Example 18 therein) as an inhibitor of ERK, and that it is useful in the treatment of cancer. WO2017/080979 A1 further describes the adipic acid co-crystal of the compound of Formula **(I)** (Example 34 therein). The compound of Formula **(I)** is also known by its chemical name: (*R*)-7-(3,4-difluorobenzyl)-6-(methoxymethyl)-2-(5-methyl-2-((1-methyl-1 *H* -pyrazol-5-yl)amino)pyrimidin-4-yl)-6,7-dihydroimidazo [1,2-a] pyrazin-8(5*H*)-one.

WO2017/080979 A1 further describes a method for the production of the compound of Formula **(I),** with the method summarised in Scheme 1 below. The following abbreviations are used in Scheme 1: SEM = 2-(trimethylsilyl)ethoxymethyl; NBS = *N*-Bromosuccinimide; pin = pinacolato; Ac = acyl, TFA = trifluoroacetic acid; 18-crown-6 = 1,4,7,10,13,16-hexaoxacyclooctadecane; and Boc = *tert*-butyloxycarbonyl.

Although the synthetic route described in the prior art provides a reliable method for producing the compound of Formula **(I)** on a laboratory scale, the route has a number of drawbacks. These include:
(i) The synthetic route is relatively long, with 10 isolated intermediates in the longest linear sequence;
(ii) The route depends on the compound of Formula **(A),** otherwise known by the chemical name *tert*-butyl (4*S*)-4-(methoxymethyl)-2,2-dioxo-oxathiazolidine-3-carboxylate, which is of high cost and limited chemical stability at room temperature; and
(iii) The compound of Formula **(I)** formed by this method required purification by chromatography, which is expensive on large scale.

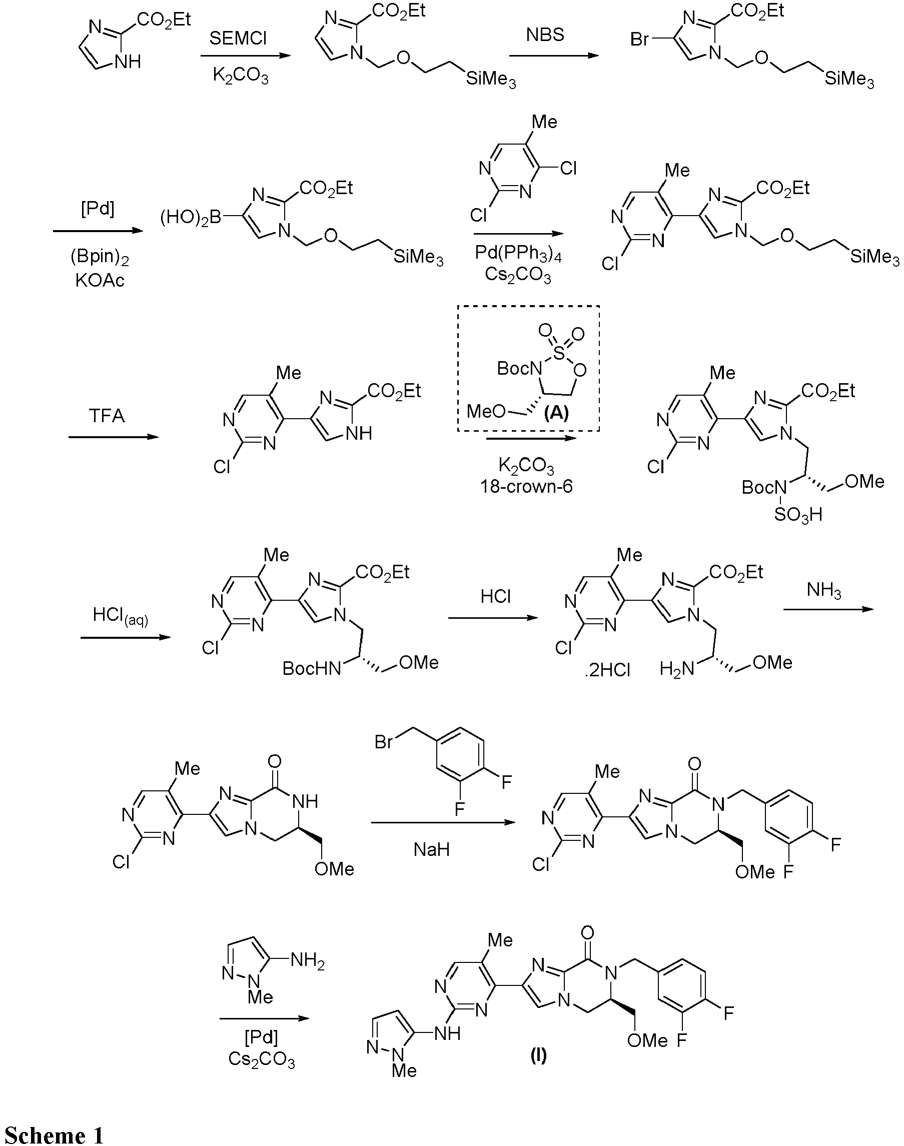

Thus, whilst the synthetic route shown in Scheme 1 is adequate to make the compound of Formula (I) on a laboratory scale, there is a need for a process suitable for the large-scale production of the compound of Formula **(I).**

We have now found an improved synthetic process which substantially overcomes the drawbacks described above. The improved process is summarised in Scheme 2 below.

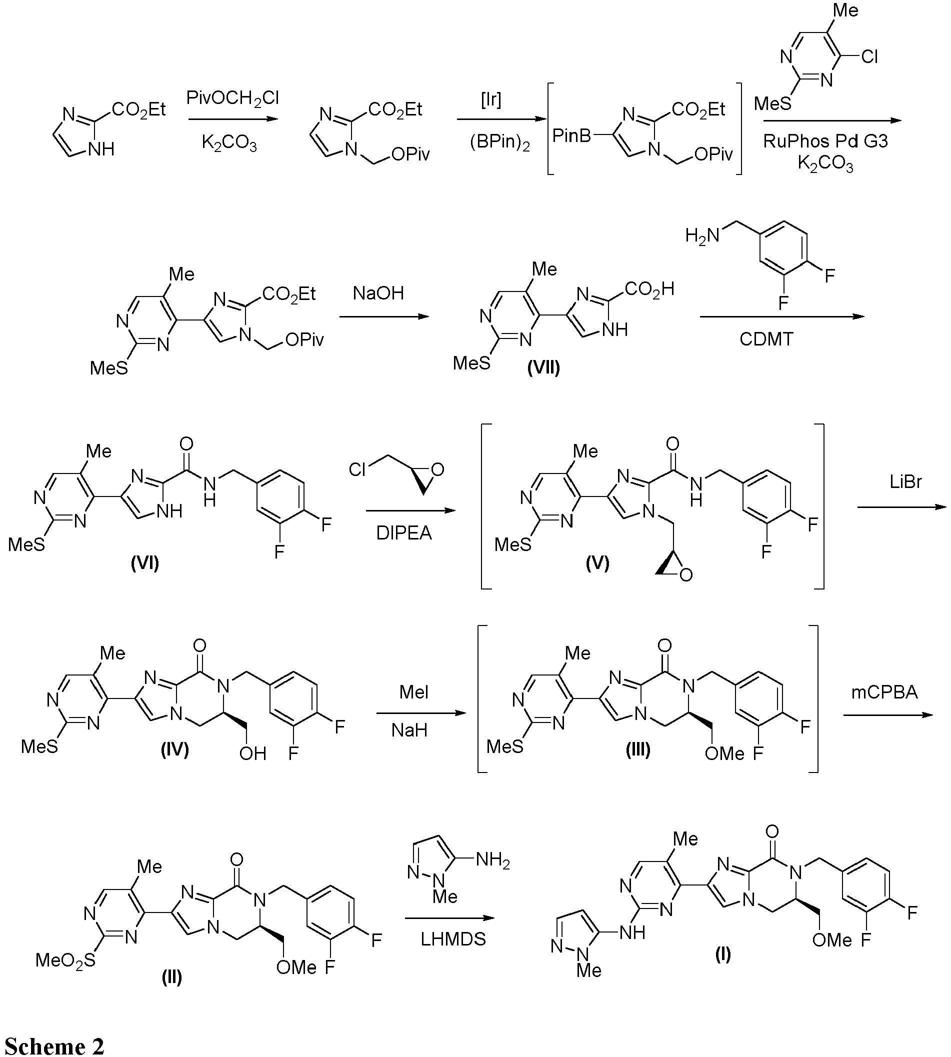

In Scheme 2 the following further abbreviations are used: Piv = pivaloyl; RuPhos Pd G3 = (2-dicyclohexylphosphino-2',6'-diisopropoxy-1,1'-biphenyl-crysta)[2-(2'-amino-1,1'-biphenyl)]palladium(II) methanesulfonate; CDMT = 2-chloro-4,6,-dimethoxy-1,3,5-triazine; DIPEA = *N*,*N-*diisopropylethylamine; mCPBA = *meta*-chloroperoxybenzoic acid; and LHMDS = lithium bis(trimethylsilyl)amide. Square brackets indicate that it was not necessary to isolate the compound during the synthetic sequence.

The improved process of Scheme 2 was shown to be suitable for manufacture of the compound of Formula **(I)** on a multikilogram scale. In particular:
(i) It is not necessary to isolate the compounds Formula **(V)** or Formula **(III),** so only 6 intermediates require isolation;
(ii) The improved process avoids the need for the compound of Formula **(A),** reducing the cost of goods; and
(iii) The compound of Formula **(I)** is formed in sufficient purity that it can be isolated by crystallisation with adipic acid to form the adipic acid co-crystal of the compound of Formula **(I),** avoiding the need for chromatography.

In the first aspect, this specification provides a process for the production of the compound of Formula **(I):** 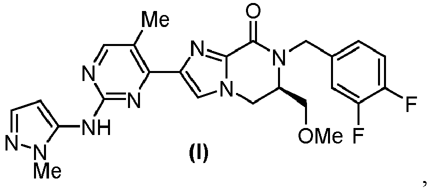 comprising a reaction of a compound of Formula **(II),** (6*R*)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one: 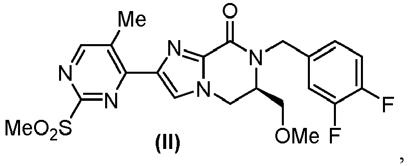 and 2-methylpyrazol-3-amine **(VIII)**

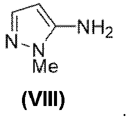

In the next aspect, this specification provides a compound of Formula **(II),** (6*R*)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one:

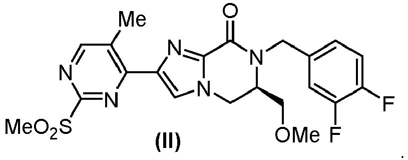

In the next aspect, this specification provides a compound of Formula **(III),** (6*R*)-7-[(3,4-difluorophenyl)methyl]-6-(methoxymethyl)-2-(5-methyl-2-methylsulfanyl-pyrimidin-4-yl)-5,6-dihydroimidazo[1,2-a]pyrazin-8-one:

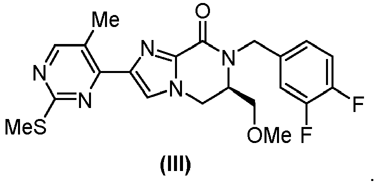

In the next aspect, this specification provides a compound of Formula **(IV),** (6*R*)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one:

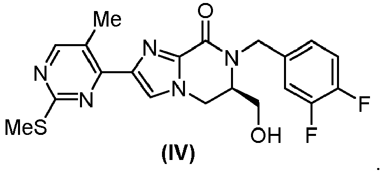

In the next aspect, this specification provides a compound of Formula **(V),** *N-*[(3,4-difluorophenyl)methyl]-4-(5-methyl-2-methylsulfanyl-pyrimidin-4-yl)-1-[[(2*R*)-oxiran-2-yl]methyl]imidazole-2-carboxamide:

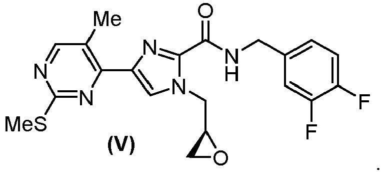

In the next aspect, this specification provides a compound of Formula **(VI),** *N-*[(3,4-difluorophenyl)methyl]-4-(5-methyl-2-methylsulfanyl-pyrimidin-4-yl)-1*H*-imidazole-2-carboxamide:

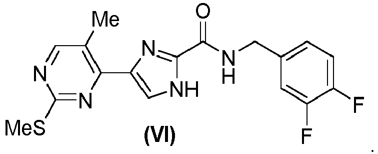

In the next aspect, this specification provides a compound of Formula **(VII),** 4-(5-methyl-2-methylsulfanyl-pyrimidin-4-yl)-1*H*-imidazole-2-carboxylic acid:

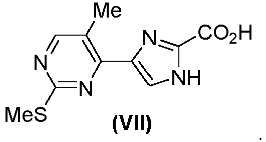

As set forth above, there is provided a process for the production of a compound of Formula **(I)** 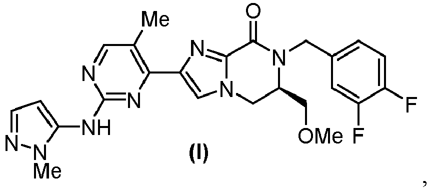 comprising the step of (i) the reaction of a compound of Formula **(II),** (6*R*)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one: 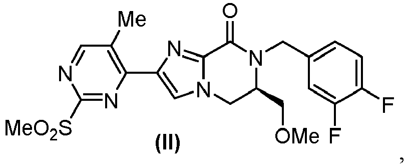 and 2-methylpyrazol-3-amine **(VIII):**

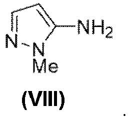

In an embodiment, the reaction is conducted in the presence of a base, such as lithium bis(trimethylsilyl)amide, sodium bis(trimethylsilyl)amide, potassium bis(trimethylsilyl)amide, potassium *tert*-butoxide or sodium hydride. In a further embodiment, the base is lithium bis(trimethylsilyl)amide.

In embodiments, the process comprises the further step of (ii) making the compound of Formula **(II)** from the reaction of a compound of Formula **(III),** (6*R*)-7-[(3,4-difluorophenyl)methyl]-6-(methoxymethyl)-2-(5-methyl-2-methylsulfanyl-pyrimidin-4-yl)-5,6-dihydroimidazo[1,2-a]pyrazin-8-one: 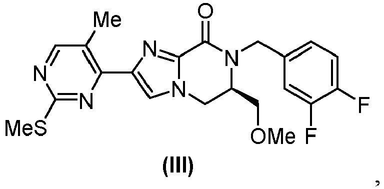 and an oxidising agent. In embodiments, the oxidising agent is hydrogen peroxide, oxone or mCPBA. In further embodiments, the oxidising agent is mCPBA.

In embodiments, the process comprises the further step of (iii) making the compound of Formula (**III**) from the reaction of a compound of Formula **(IV),** (6*R*)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one: 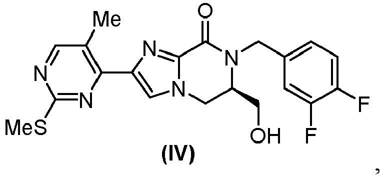 and a methylating agent, such as methyl iodide, dimethyl sulfate or methyl triflate. In further embodiments, the methylating agent is methyl iodide. In further embodiments, the reaction is conducted in the presence of a base, such as sodium hydride, lithium bis(trimethylsilyl)amide, sodium bis(trimethylsilyl)amide, potassium bis(trimethylsilyl)amide or potassium *tert*-butoxide. In further embodiments, the base is sodium hydride.

In embodiments, the process comprises the further step of (iv) making the compound of Formula **(IV)** from the reaction of a compound of Formula **(V),** *N-*[(3,4-difluorophenyl)methyl]-4-(5-methyl-2-methylsulfanyl-pyrimidin-4-yl)-1-[[(2*R*)-oxiran-2-yl]methyl]imidazole-2-carboxamide: 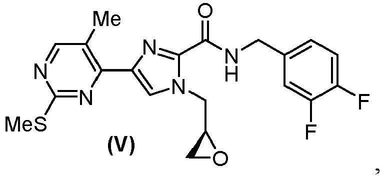 and lithium bromide.

In embodiments, the process comprises the further step of (v) making the compound of Formula **(V)** from the reaction of a compound of Formula **(VI),** *N-*[(3,4-difluorophenyl)methyl]-4-(5-methyl-2-methylsulfanyl-pyrimidin-4-yl)-1*H*-imidazole-2-carboxamide: 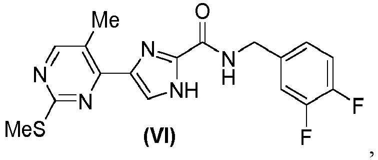 and (*S*)-(+)-epichlorohydrin **(IX):**

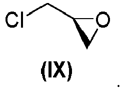

In embodiments, the reaction is conducted in the presence of 4-dimethylaminopyridine. In further embodiments, the reaction is conducted in the presence of a base, such as DIPEA, triethylamine or tributylamine. In further embodiments, the base is DIPEA.

In embodiments, the process comprises the further step of (vi) making the compound of Formula **(VI)** from the reaction of a compound of Formula **(VII),** 4-(5-methyl-2-methylsulfanyl-pyrimidin-4-yl)-1*H*-imidazole-2-carboxylic acid: 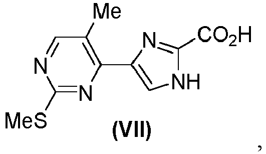 and (3,4-difluorophenyl)methanamine **(X):**

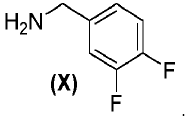

In further embodiments, the reaction is conducted in the presence of a peptide coupling agent, such as 2-chloro-4,6-dimethoxy-1,3,5-triazine in combination with 4-methylmorpholine, 1-ethyl-3-(3-dimethylaminopropyl)carbodiimide in combination with hydroxybenzotriazole, benzotriazol-1-yl-oxytripyrrolidinophosphonium hexafluorophosphate, propanephosphonic acid anhydride, O-(benzotriazol-1-yl)-N,N,N',N'-tetramethyluronium tetrafluoroborate, 1,1'-carbonyldiimidazole, an acid halide forming reagent or an acid anhydride forming reagent. In further embodiments, the peptide coupling agent is 2-chloro-4,6-dimethoxy-1,3,5-triazine in combination with 4-methylmorpholine.

In an embodiment, there is provided a compound of Formula **(II),** (6*R*)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one:

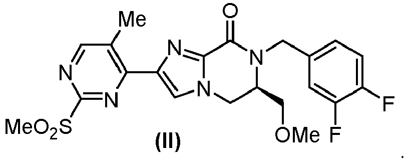

In a further embodiment, the compound of Formula **(II)** is in an enantiomeric excess (%ee) of ≥ 95%, ≥ 98% or ≥ 99%. In a further embodiment, the compound of Formula **(II)** is in an enantiomeric excess (%ee) of ≥ 99%.

In an embodiment, there is provided a compound of Formula **(III),** (6*R*)-7-[(3,4-difluorophenyl)methyl]-6-(methoxymethyl)-2-(5-methyl-2-methylsulfanyl-pyrimidin-4-yl)-5,6-dihydroimidazo[1,2-a]pyrazin-8-one:

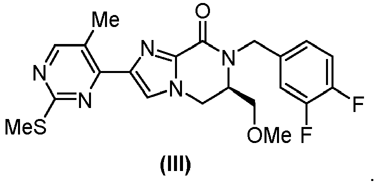

In a further embodiment, the compound of Formula **(III)** is in an enantiomeric excess (%ee) of ≥ 95%, ≥ 98% or ≥ 99%. In a further embodiment, the compound of Formula **(III)** is in an enantiomeric excess (%ee) of ≥ 99%.

In an embodiment, there is provided a compound of Formula **(IV),** (6*R*)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one:

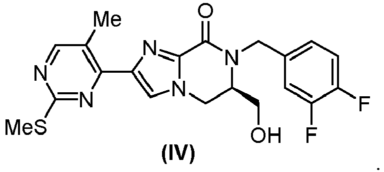

In a further embodiment, the compound of Formula **(IV)** is in an enantiomeric excess (%ee) of ≥ 95%, ≥ 98% or ≥ 99%. In a further embodiment, the compound of Formula **(IV)** is in an enantiomeric excess (%ee) of ≥ 99%.

In an embodiment, there is provided a compound of Formula **(V),** *N-*[(3,4-difluorophenyl)methyl]-4-(5-methyl-2-methylsulfanyl-pyrimidin-4-yl)-1-[[(2*R*)-oxiran-2-yl]methyl]imidazole-2-carboxamide:

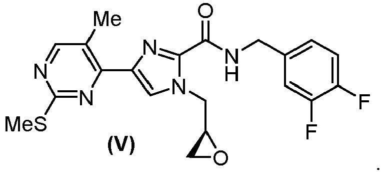

In a further embodiment, the compound of Formula **(V)** is in an enantiomeric excess (%ee) of ≥ 95%, ≥ 98% or ≥ 99%. In a further embodiment, the compound of Formula **(V)** is in an enantiomeric excess (%ee) of ≥ 99%.

In an embodiment, there is provided a compound of Formula **(VI),** *N-*[(3,4-difluorophenyl)methyl]-4-(5-methyl-2-methylsulfanyl-pyrimidin-4-yl)-1*H*-imidazole-2-carboxamide:

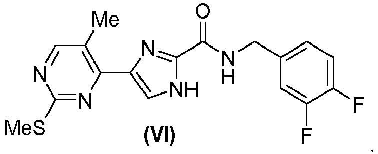

In an embodiment, there is provided a compound of Formula **(VII),** 4-(5-methyl-2-methylsulfanyl-pyrimidin-4-yl)-1*H*-imidazole-2-carboxylic acid:

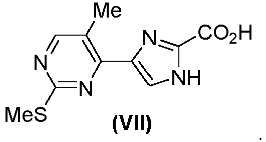

Compounds described in this specification may form acid addition salts or base addition salts.

In general, an acid addition salt can be prepared using various inorganic or organic acids. Such salts can typically be formed by, for example, mixing the compound with an acid (e.g., a stoichiometric amount of acid) using various methods known in the art. This mixing may occur in water, an organic solvent (e.g., ether, ethyl acetate, ethanol, isopropanol, or acetonitrile), or an aqueous/organic mixture. An acid addition salt may for example be formed using an inorganic acid selected from the group consisting of hydrochloric acid.

For compounds that may form base addition salts, it may be possible to make, for example, an alkali metal (such as sodium, potassium, or lithium) or an alkaline earth metal (such as a calcium) salt by treating a compound with an alkali metal or alkaline earth metal hydroxide or alkoxide (e.g., an ethoxide or methoxide) or a suitably basic organic amine (e.g., a choline or meglumine) in an aqueous medium.

The general principles and techniques of preparing salts can be found in Berge et al., J. Pharm. Sci., 66, 1-19 (1977).

Compounds described in this specification may exist in solvated forms and unsolvated forms. For example, a solvated form may be a hydrated form, such as a hemi-hydrate, a mono-hydrate, a di-hydrate, a tri-hydrate or an alternative quantity thereof. This specification encompasses all such solvated and unsolvated forms.

Atoms of the compounds and salts described in this specification may exist as their isotopes. This specification encompasses all such compounds where an atom is replaced by one or more of its isotopes (for example a compound where one or more carbon atom is an ¹¹C or ¹³C carbon isotope, or where one or more hydrogen atoms is a ²H or ³H isotope).

The compound of Formula **(I),** prepared by the processes described herein, may be used to provide formulations, such as tablets, for use as medicaments for the treatment of cancer. Suitable formulations and therapeutic uses of the medicaments so prepared are described in WO2017/080979 A1.

The various embodiments are illustrated by the following Examples. The disclosure is not to be interpreted as being limited to the Examples. During the preparation of the Examples, generally:
i. Operations were carried out at ambient temperature, i.e. in the range of about 17 to 30 °C and under an atmosphere of an inert gas such as nitrogen unless otherwise stated;
ii. Yields, where present, are not necessarily the maximum attainable;
iii. Compounds were confirmed by nuclear magnetic resonance (NMR) spectroscopy, with NMR chemical shift values measured on the delta scale. Proton magnetic resonance spectra were determined using a Bruker advance 700 (700MHz), Bruker Avance 500 (500 MHz), Bruker 400 (400 MHz) or Bruker 300 (300 MHz) instrument; measurements were taken at around 20 - 30°C unless otherwise specified; the following abbreviations have been used: s = singlet; d = doublet; t = triplet; q = quartet; p = pentet/quintet; m = multiplet; dd = doublet of doublets; ddd = doublet of doublet of doublet; dt = doublet of triplets; td = triplet of doublets; qd = quartet of doublets; bs = broad signal;
iv. Compounds were also characterised by high resolution mass spectrometry following liquid chromatography (LC-MS). LC-MS was carried out using a Waters H-Class Bio UPLC connected to a Waters Synapt G2-Si ESI mass spectrometer operating in positive electrospray mode and an Acquity BEH RP18 column (2.1 × 100mm, 1.7mm) at a flow rate of 0.6 mL/min using a solvent system of 90/5/5 A/B/C to 5/90/5 A/B/C over 12 minutes followed by a hold at 5/90/5 A/B/C for 1.2 minutes, where A = water, B = acetonitrile and C = 250mM ammonium acetate in water;
v. IUPAC names were generated using BIOVIA^{™} Draw, version 16.1.
vi. The following abbreviations have been used: CDMT = 2-chloro-4,6-dimethoxy-1,3,5-triazine; CPME= cyclopropyl methyl ether; DIPEA = *N*,*N-*diisopropylethylamine; DMF = Dimethylformamide; LHMDS = lithium bis(trimethylsilyl)amide; mCPBA = meta-chloroperoxybenzoic acid; Piv = pivaloyl; and RuPhos Pd G3 = (2-dicyclohexylphosphino-2',6'-diisopropoxy-1,1'-biphenyl)[2-(2'-amino-1,1 '-biphenyl)]palladium(II) methanesulfonate.
vii. (6*R*)-7-[(3,4-Difluorophenyl)methyl]-6-(methoxymethyl)-2-[5-methyl-2-[(2-methylpyrazol-3-yl)amino]pyrimidin-4-yl]-5,6-dihydroimidazo[1,2-a]pyrazin-8-one adipic acid co-crystal seed corresponds to Example 34 of WO2017/080979 A1 (page 180 therein) and can be prepared according to the method disclosed therein.

### Ethyl 1-(2,2-dimethylpropanoyloxymethyl)imidazole-2-carboxylate

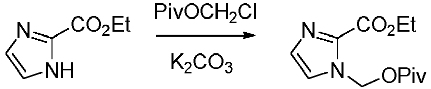

Chloromethyl pivalate (632.5 mmol, 95.25 g, 91.15 mL) was added dropwise over 2 hours to a stirring mixture of ethyl 1H-imidazole-2-carboxylate (80.58 g, 575.0 mmol), potassium carbonate (690.0 mmol, 95.36 g) and acetonitrile (730 mL) at 40 °C. The resulting mixture was then stirred for 20 hours at 40 °C. This was then cooled to 20 °C and filtered, washing with acetonitrile (160 mL). The resulting filtrate was concentrated under vacuum. Residual acetonitrile was removed by three cycles of treating the residue with heptane and then concentrating the resulting mixture under vacuum. The resulting suspension was then filtered, washing with heptane (160 mL), to yield the title compound as an off-white solid (134 g, 92%); ¹H NMR (500 MHz, DMSO-d6) δ ppm 1.1 (s, 9 H) 1.3 (t, J=7.1 Hz, 3 H) 4.3 (q, J=7.1 Hz, 2 H) 6.2 (s, 2 H) 7.1 (d, J=1.1 Hz, 1 H) 7.6 (d, J=1.1 Hz, 1 H); m/z = 254.28.

### Ethyl 1-(2,2-dimethylpropanoyloxymethyl)-4-(5-methyl-2-methylsulfanyl-pyrimidin-4-yl)imidazole-2-carboxylate

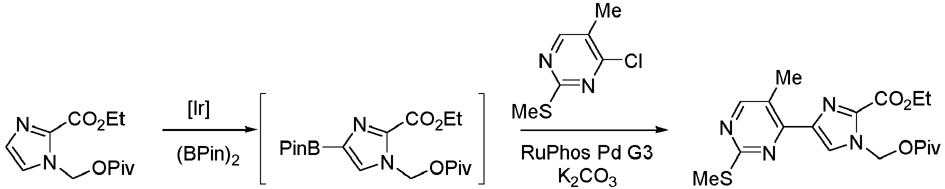

(1,5-Cyclooctadiene)(methoxy)iridium (I) dimer (2.06 mmol, 1.37 g) was added to a stirring mixture of ethyl 1-(2,2-dimethylpropanoyloxymethyl)imidazole-2-carboxylate (138 mmol, 35.0 g), bis(pinacolato)diboron (145 mmol, 36.7 g) and 3,4,7,8-tetramethyl-1,10-phenanthroline (4.13 mmol, 0.976 g) in CPME (140 mL) and the resulting mixture was stirred at 53 °C for 1 hour. The resulting mixture was then filtered, washing with CPME (35 mL), to yield crude intermediate ethyl 1-(2,2-dimethylpropanoyloxymethyl)-4-methyl-imidazole-2-carboxylate.

RuPhos Pd G3 (3.44 mmol, 2.88 g) was added to a mixture of 4-chloro-5-methyl-2-(methylsulfanyl)pyrimidine (138 mmol, 24.0 g), potassium carbonate (275 mmol, 38.0 g), CPME (280 mL) and water (140 mL) and the resulting mixture stirred at 55 °C. The crude intermediate ethyl 1-(2,2-dimethylpropanoyloxymethyl)-4-methyl-imidazole-2-carboxylate was added to the resulting mixture, which was then stirred at 55 °C for 1 hour. The mixture was then filtered through CELITE^{™} (35.0 g) at 40 °C, washing with CPME (35 mL). The aqueous layer of the resulting filtrate was removed, and the organic layer was washed with water (140 mL). The organic layer was then concentrated under vacuum and cooled to 5 °C to yield a solid which was filtered, washing with CPME (35 mL), to yield the title compound as a light pink solid (54.0 g, 138 mmol); ¹H NMR (500 MHz, CHLOROFORM-d) δ ppm 1.0 (s, 9 H) 1.5 (t, J=7.1 Hz, 3 H) 2.3 (d, J=0.5 Hz, 3 H) 2.6 (s, 3 H) 4.5 (q, J=7.1 Hz, 2 H) 6.7 (s, 2 H) 7.5 (s, 1 H) 8.5 - 8.5 (m, 1 H); m/z = 392.47.

### N-[(3,4-difluorophenyl)methyl]-4-(5-methyl-2-methylsulfanyl-pyrimidin-4-yl)-1H-imidazole-2-carboxamide

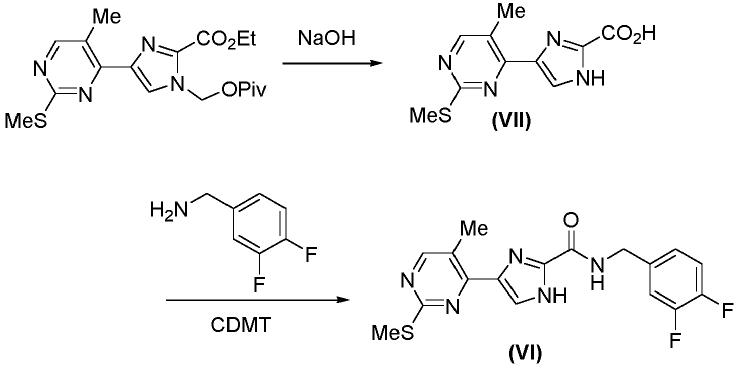

An aqueous solution of sodium hydroxide (2.0 M, 441.2 mmol, 220.6 mL) was added dropwise over 15 minutes to a stirring mixture of ethyl 1-(2,2-dimethylpropanoyloxymethyl)-4-(5-methyl-2-methylsulfanyl-pyrimidin-4-yl)imidazole-2-carboxylate (55.07 g, 140.3 mmol) in THF (330 mL) and water (550 mL) at 15 °C. The mixture was stirred at 15 °C for 12 hours before a solution of phosphoric acid (646 mmol, 85 mass%, 74.5 g, 44.06 mL) in water (275 mL) was added dropwise over 15 minutes at 15 °C. After a further 10 minutes the mixture was filtered, washing with water (275 mL), to yield intermediate 4-(5-methyl-2-methylsulfanyl-pyrimidin-4-yl)-1H-imidazole-2-carboxylic acid; 1H NMR (400 MHz, DMSO-d6) δ ppm 2.5 (s, 3 H) 2.6 (s, 3 H) 8.0 (s, 1 H) 8.5 (s, 1 H); m/z = 250.28.

The intermediate was dissolved in DMF (720 mL) and cooled to 0 °C, then treated with CDMT (210.5 mmol, 36.95 g). The reaction mixture was stirred for 10 minutes at 0 °C, before being treated dropwise over 15 minutes with a solution of (3,4-difluorophenyl)methanamine (168.4 mmol, 24.10 g, 19.9 mL) and 4-methylmorpholine (168.4 mmol, 24.10 g, 19.9 mL) in DMF (83 mL) pre-cooled to 15 °C, with the reaction mixture held at 0 °C. The reaction mixture was stirred at 0 °C for 2 hours, then treated with water (250 mL) over 10 minutes at 5 °C. The resulting mixture was then filtered, washing with a mixture of DMF (55 mL) and water (55 mL). The resulting filter cake was dried under vacuum and then charged with methanol (275 mL) at 20 °C. The resulting slurry was stirred and treated with a solution of potassium carbonate (199.2 mmol, 27.54 g) in water (253 mL) at 20 °C. The mixture was stirred at 20 °C for 12 hours before being filtered and sequentially washed with water (275 mL) and methanol (275 mL). The filtered solid was dried under vacuum to give the title compound as a solid (42.9 g, 114 mmol); 1H NMR (500 MHz, DMSO-d6) δ ppm 2.5 - 2.5 (m, 3 H) 2.6 (br s, 3 H) 4.5 (d, J=6.4 Hz, 2 H) 7.1 - 7.2 (m, 1 H) 7.3 - 7.5 (m, 2 H) 7.7 - 8.2 (m, 1 H) 8.4 (s, 1 H) 9.0 (br s, 1 H) 13.6 (br s, 1 H); 19F NMR (470 MHz, DMSO-d6) δ ppm -141.7 - -141.4 (m, 1 F) - 139.1 - -138.9 (m, 1 F); 13C NMR (126 MHz, DMSO-d6) δ ppm 13.5 (s, 1 C) 16.8 (s, 1 C) 41.3 (s, 1 C) 116.3 (d, J=17.3 Hz, 1 C) 117.2 (d, J=16.8 Hz, 1 C) 121.9 (s, 1 C) 123.1 (s, 1 C) 124.0 (dd, J=6.6, 3.4 Hz, 1 C) 137.3 (dd, J=4.3 Hz, 1 C) 140.3 (s, 1 C) 141.2 (s, 1 C) 148.4 (dd, J=244.1, 12.5 Hz, 1 C) 149.2 (dd, J=245.2, 12.7 Hz, 1 C) 156.8 (s, 1 C) 158.3 (s, 1 C) 159.7 (s, 1 C) 167.7 (s, 1 C); m/z = 375.4.

### (6R)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one

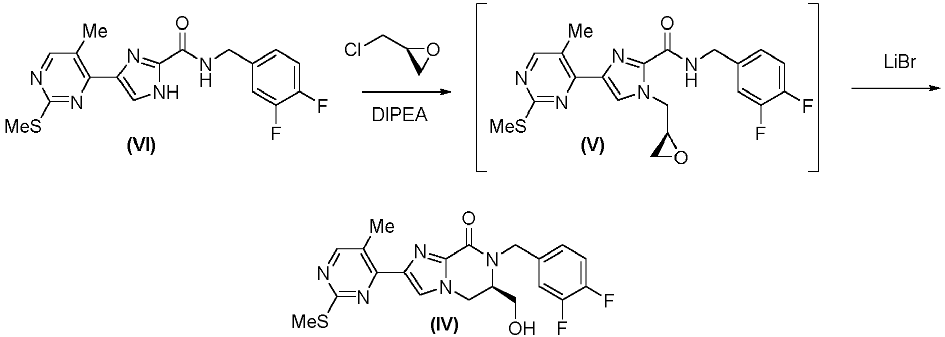

A mixture of *N*-[(3,4-difluorophenyl)methyl]-4-(5-methyl-2-methylsulfanyl-pyrimidin-4-yl)-1*H*-imidazole-2-carboxamide (115.8 mmol, 43.47 g), 4-dimethylaminopyridine (3.474 mmol, 0.4244 g), DIPEA (115.8 mmol, 14.97 g, 20.2 mL) and (*S*)-(+)-epichlorohydrin (926.4 mmol, 85.71 g, 72.51 mL) in THF (535 mL) was stirred at 20 °C before being heated to 50 °C over 1 hour. The resulting mixture was then heated at 50 °C for 24 hours to give a slurry of *N*-[(3,4-difluorophenyl)methyl]-4-(5-methyl-2-methylsulfanyl-pyrimidin-4-yl)-1-[[(2*R*)-oxiran-2-yl]methyl]imidazole-2-carboxamide. This was then cooled to 30 °C and treated with lithium bromide (162.1 mmol, 14.08 g) portionwise over 10 minutes at 30 °C. The mixture was then stirred at 40 °C for 16 hours, before being cooled to 20 °C. The mixture was then filtered, washing with THF (87 mL). The filtered solid was then stirred in methanol (430 mL) at 20 °C for 1 hour, before the mixture was filtered, washing with methanol (87 mL). The filter cake was dried under vacuum to give the title compound as a solid (49.97 g, 115.8 mmol); ¹H NMR (500 MHz, DMSO-d6) δ ppm 2.5 (s, 3 H) 2.5 - 2.6 (m, 3 H) 3.2 - 3.3 (m, 1 H) 3.4 - 3.5 (m, 1 H) 3.8 - 3.8 (m, 1 H) 4.4 (d, J=15.5 Hz, 1 H) 4.4 (dd,J=13.6, 4.9 Hz, 1 H) 4.5 - 4.5 (m, 1 H) 5.1 (d, J=15.5 Hz, 1 H) 5.2 (t, J=5.4 Hz, 1 H) 7.2 - 7.3 (m, 1 H) 7.4 (dt, J=10.8, 8.5 Hz, 1 H) 7.5 (ddd, J=11.6, 7.9, 2.0 Hz, 1 H) 8.2 (s, 1 H) 8.5 (d, J=0.6 Hz, 1 H); m/z = 431.46.

### (6R)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one

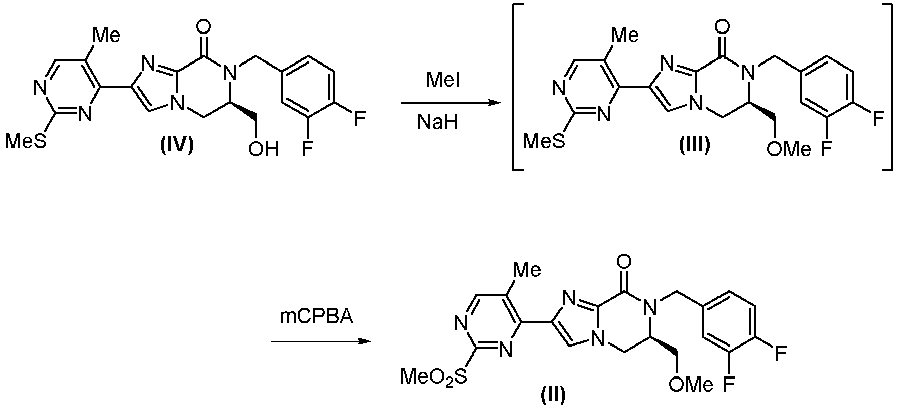

A mixture of (6*R*)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one (61.2 g, 142 mmol) in THF (551 mL) was stirred at 20 °C for 15 minutes, before being cooled to 0 °C. The mixture was then treated with sodium hydride (60 mass% in mineral oil, 170 mmol, 6.81 g) portionwise over 20 minutes at 0 °C. The mixture was stirred at 0 °C for 1 hour and then treated with iodomethane (213 mmol, 30.2 g, 13.3 mL) dropwise over 10 minutes at 0 °C. The mixture was treated with THF (61 mL) as a line wash, then stirred for 30 minutes at 0 °C, then warmed to 35 °C over 1 hour and then stirred at 35 °C for 1 hour. The mixture was then cooled to 20 °C and treated with a solution of sodium chloride (314 mmol, 18.4 g) in water (122 mL). The mixture was stirred at 20 °C for 10 minutes before the aqueous layer was removed. The organic layer was stirred and treated with a solution of sodium chloride (314 mmol, 18.4 g) in water (122 mL). The mixture was stirred at 20 °C for 10 minutes before the aqueous layer was removed. The organic layer was concentrated under vacuum, then treated with dichloromethane (612 mL) and water (122 mL). The resulting mixture was stirred at 20 °C for 10 minutes before the aqueous layer was removed, to give a solution of (6*R*)-7-[(3,4-difluorophenyl)methyl]-6-(methoxymethyl)-2-(5-methyl-2-methylsulfanyl-pyrimidin-4-yl)-5,6-dihydroimidazo[1,2-a]pyrazin-8-one [1H NMR (400 MHz, Chloroform-d) δ ppm 2.6 (s, 3 H) 2.7 (s, 3 H) 3.3 (s, 3 H) 3.4 (m, 1 H) 3.7 (m, 1 H) 3.8 (m, 1 H) 4.2 (m, 2 H) 4.4 (d, 1 H) 5.4 (d, 1 H) 7.1 - 7.2 (m, 3 H) 7.9 (s, 1 H) 8.4 (s, 1 H); m/z = 445.49]. This was cooled to 5 °C and treated portionwise with mCPBA (312 mmol, 77 mass%, 69.9 g) over 20 minutes at 5 °C. The mixture was stirred at 5 °C for 1 hour, then warmed to 20 °C over 30 minutes, then stirred at 20 °C for 10 hours. The mixture was then treated with a solution of sodium sulfite (486 mmol, 61.2 g) in water (282 mL) and stirred for a further 10 minutes at 20 °C. The aqueous layer was then removed, and the organic layer treated with a solution of potassium carbonate (221 mmol, 30.6 g) in water (282 mL). The mixture was stirred for 10 minutes before the aqueous layer was removed, and the organic layer treated with a solution of potassium carbonate (221 mmol, 30.6 g) in water (282 mL). The mixture was stirred for 10 minutes before the aqueous layer was removed, and the organic layer treated portionwise with magnesium sulfate (153 mmol, 18.4 g). The mixture was stirred for 10 minutes before being filtered through CELITE^{™} (18.4 g), washing with dichloromethane (245 mL). The filtrate was concentrated under vacuum, then added to diisopropyl ether (918 mL) at 20 °C over 20 minutes. Dichloromethane (12.2 mL) was used to wash residual filtrate into the mixture. The mixture was then stirred for 3 hours at 20 °C then filtered, washing with diisopropyl ether (122 mL). The filter cake was dried under vacuum to give the title compound (67.7 g, 142 mmol) as a solid; ¹H NMR (500 MHz, DMSO-d6) δ ppm 2.7 (s, 3 H) 3.2 (s, 3 H) 3.3 - 3.4 (m, 1 H) 3.4 (dd, J=10.1, 4.8 Hz, 1 H) 3.4 - 3.4 (m, 3 H) 4.0 - 4.1 (m, 1 H) 4.4 (d,J=15.6 Hz, 1 H) 4.5 (d, J=3.0 Hz, 2 H) 5.1 (d, J=15.6 Hz, 1 H) 7.2 - 7.3 (m, 1 H) 7.4 (dt, J=10.7, 8.5 Hz, 1 H) 7.4 - 7.5 (m, 1 H) 8.4 (s, 1 H) 8.9 (s, 1 H); ¹³C NMR (126 MHz, DMSO-d6) δ ppm 17.5 (s, 1 C) 39.0 (s, 1 C) 44.3 (s, 1 C) 47.5 (s, 1 C) 54.3 (s, 1 C) 58.6 (s, 1 C) 70.8 (s, 1 C) 116.7 (d, J=17.3 Hz, 1C) 117.4 (d, J=17.3 Hz, 1 C) 124.5 (dd, J=6.4, 3.6 Hz, 1 C) 126.3 (s, 1 C) 129.6 (s, 1 C) 135.5 (s, 1 C) 138.4 (s, 1 C) 139.9 (s, 1 C) 148.6 (dd, J=244.8,12.3 Hz, 1 C) 149.4 (dd, J=245.5, 12.9 Hz, 1 C) 155.6 (s, 1 C) 157.4 (s, 1 C) 160.8 (s, 1 C) 163.1 (s, 1 C); ¹⁹F NMR (470 MHz, DMSO-d6) δ ppm -140.9 (d, J=22.1 Hz, 1 F) -138.6 (d, J=22.1 Hz, 1 F); m/z = 477.48.

### (6R)-7-[(3,4-difluorophenyl)methyl]-6-(methoxymethyl)-2-[5-methyl-2-[(2-methylpyrazol-3-yl)amino]pyrimidin-4-yl]-5,6-dihydroimidazo[1,2-a]pyrazin-8-one, and the adipic acid co-crystal thereof

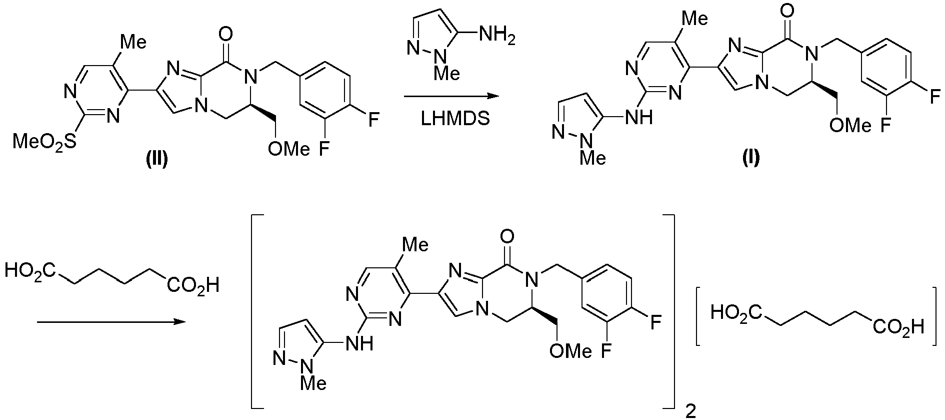

A mixture of (6R)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one (35.0 g, 73.3 mmol) and 2-methylpyrazol-3-amine (21.4 g, 220 mmol) in THF (875 mL) was stirred at 20 °C. The mixture was then concentrated by distillation at atmospheric pressure. Further THF (175 mL) was added to the mixture, and the mixture was again concentrated by distillation at atmospheric pressure. A Karl-Fisher analysis confirmed that the water content of the mixture was below 400 ppm. The mixture was then cooled to -10 °C and treated with a pre-formed mixture of LHMDS (1 M in THF) and THF (220 mL) over 30 minutes at -10 °C. THF (17.5 mL) was added to the mixture as a line wash. The mixture was stirred for 45 minutes at -10 °C before being treated with a solution of phosphoric acid (366 mmol, 85 mass%, 42.3 g) in water (131 mL) over 15 minutes. The mixture was stirred for a further 10 minutes at 15 °C, and then the aqueous layer was removed. The organic layer was treated with a solution of sodium chloride (427 mmol, 25.0 g) and phosphoric acid (366 mmol, 85 mass%, 42.3 g) in water (150 mL) at 15 °C. The mixture was stirred for 10 minutes at 15 °C and the aqueous layer removed. The organic layer was treated with a solution of sodium chloride (427 mmol, 25.0 g) in water (150 mL) and stirred for 10 minutes at minutes at 15 °C. The aqueous layer was removed, and the organic layer treated with a solution of sodium bicarbonate (12.0 g) in water (169 mL) over 15 minutes at 15 °C. The mixture was stirred for a further 10 minutes at 15 °C and the aqueous layer removed. The organic layer was concentrated by atmospheric distillation. The residue was treated with ethanol (599 mL) and the resulting mixture concentrated by atmospheric distillation to give crude product (6*R*)-7-[(3,4-difluorophenyl)methyl]-6-(methoxymethyl)-2-[5-methyl-2-[(2-methylpyrazol-3-yl)amino]pyrimidin-4-yl]-5,6-dihydroimidazo[1,2-a]pyrazin-8-one.

The crude product was treated with ethanol (7 mL) and the resulting mixture heated to 70 °C to give a crude product solution. Adipic acid (0.510 equivalents, 37.4 mmol, 100 mass%, 5.46 g) was treated with ethanol (70 mL) and heated to 30 °C until a solution was achieved. Then 30% of the adipic acid solution was added to the crude product solution over 15 minutes, with the crude product solution held at 70 °C. The crude product solution was then cooled to 55 °C and charged with (6*R*)-7-[(3,4-difluorophenyl)methyl]-6-(methoxymethyl)-2-[5-methyl-2-[(2-methylpyrazol-3-yl)amino]pyrimidin-4-yl]-5,6-dihydroimidazo[1,2-a]pyrazin-8-one adipic acid co-crystal seed (3.09 mmol, 1.75 g). The resulting mixture was held at 55 °C for 1 hour. The remaining adipic acid solution was added to the crude product solution dropwise over 3 hours using ethanol (7 mL) as a line rinse, and with the crude product solution held at 55 °C. The resulting mixture was cooled to 5 °C over 3 hours, and then held at 5 °C for a further 3 hours. The resulting mixture was filtered, washing with ethanol (70 mL). The solid was then stirred with n-heptane (175 mL) at 20 °C for 1 hour and filtered again, washing with n-heptane (70 mL). The solid was then dried under vacuum to give (6R)-7-[(3,4-difluorophenyl)methyl]-6-(methoxymethyl)-2-[5-methyl-2-[(2-methylpyrazol-3-yl)amino]pyrimidin-4-yl]-5,6-dihydroimidazo[1,2-a]pyrazin-8-one adipic acid co-crystal as a solid (41.6 g, 73.3 mmol) as a solid. 1H NMR (500 MHz, METHANOL-d4) d ppm 1.5 - 1.7 (m, 4 H) 2.2 - 2.4 (m, 4 H) 2.5 (s, 6 H) 3.2 (s, 6 H) 3.4 (dd, J=9.4, 7.2 Hz, 2 H) 3.5 (dd, J=10.0, 4.6 Hz, 2H) 3.7 (s, 6 H) 3.9 - 4.1 (m, 2 H) 4.4 (dd, J=13.2, 5.1 Hz, 2 H) 4.5 (d, J=15.2 Hz, 2 H) 4.5 (dd, J=13.6, 1.1 Hz, 2 H) 5.2 (d, J=15.2 Hz, 2 H) 6.3 (d, J=2.0 Hz, 2H) 7.2 - 7.2 (m, 2 H) 7.2 - 7.3 (m, 2 H) 7.4 (ddd, J=11.3, 7.7, 1.6 Hz, 2 H) 7.4 (d, J=2.0 Hz, 2 H) 7.8 (s, 2 H) 8.2 (s, 2 H); 19F NMR (470 MHz, METHANOL-d4) d ppm -142.0 (d, J=20.4 Hz, 2 F) -139.9 (d, J=20.4 Hz, 2 F); 13C NMR (126 MHz, METHANOL-d4) d ppm 17.0 (s, 1 C) 25.5 (s, 2 C) 34.6 (s, 2 C) 35.6 (s, 2 C) 45.7 (s, 1 C) 56.4 (s, 2 C) 59.5 (s, 2 C) 72.3 (s, 2 C) 100.1 (s, 2 C) 118.2 (d, J=18.2 Hz, 2 C) 118.4 - 118.8 (m, 2 C) 120.2 (s, 2 C) 125.2 (s, 2 C) 125.7 (dd, J=6.6, 3.9 Hz, 4 C) 136.2 (s, 1 C) 139.1 (s, 2 C) 139.2 (s, 2 C) 140.6 (s, 2 C) 143.6 (s, 2 C) 151.2 (dd, J=247.5, 12.7 Hz, 2 C) 151.6 (dd, J=247.0, 12.7 Hz, 2 C) 158.3 (s, 2 C) 159.0 (s, 2 C) 160.0 (s, 2 C) 161.7 (s, 2 C) 177.3 (s, 2 C); m/z = 567.57 (free base = 494.5)

## Claims

1. A process for the production of a compound of Formula **(I):** 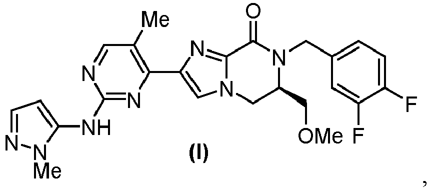 comprising the step of (i) the reaction of a compound of Formula **(II),** (6*R*)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one: 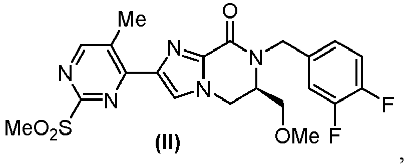 and 2-methylpyrazol-3-amine **(VIII):**

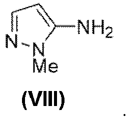

2. A process as claimed in claim 1, comprising the further step of (ii) making the compound of Formula **(II)** from the reaction of a compound of Formula **(III),** (6*R*)-7-[(3,4-difluorophenyl)methyl]-6-(methoxymethyl)-2-(5-methyl-2-methylsulfanyl-pyrimidin-4-yl)-5,6-dihydroimidazo[1,2-a]pyrazin-8-one: 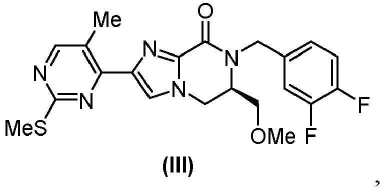 and an oxidising agent.

3. A process as claimed in claim 2, comprising the further step of (iii) making the compound of Formula **(III)** from the reaction of a compound of Formula **(IV),** (6*R*)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one: 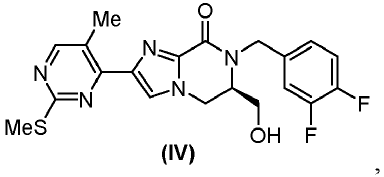 and a methylating agent.

4. A process as claimed in claim 3, comprising the further step of (iv) making the compound of Formula **(IV)** from the reaction of a compound of Formula **(V),** *N*-[(3,4-difluorophenyl)methyl]-4-(5-methyl-2-methylsulfanyl-pyrimidin-4-yl)-1-[[(2*R*)-oxiran-2-yl]methyl]imidazole-2-carboxamide: 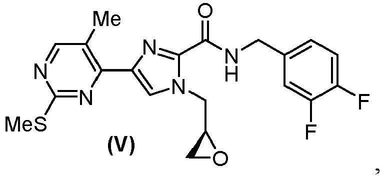 and lithium bromide.

5. A process as claimed in claim 4, comprising the further step of (v) making the compound of Formula **(V)** from the reaction of a compound of Formula **(VI),** *N*-[(3,4-difluorophenyl)methyl]-4-(5-methyl-2-methylsulfanyl-pyrimidin-4-yl)-1*H*-imidazole-2-carboxamide: 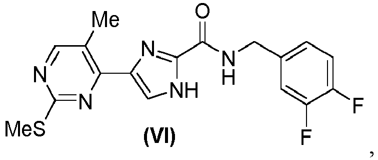 and (*S*)-(+)-epichlorohydrin **(IX):**

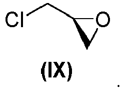

6. A process as claimed in claim 5, comprising the further step of (vi) making the compound of Formula **(VI)** from the reaction of a compound of Formula **(VII),** 4-(5-methyl-2-methylsulfanyl-pyrimidin-4-yl)-1*H*-imidazole-2-carboxylic acid: 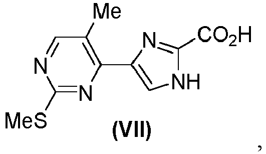 and (3,4-difluorophenyl)methanamine **(X):**

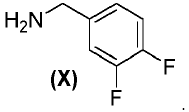

7. A compound of Formula **(II),** (6*R*)-7-[(3,4-difluorophenyl)methyl]-6-(methoxymethyl)-2-(5-methyl-2-methylsulfonyl-pyrimidin-4-yl)-5,6-dihydroimidazo[1,2-a]pyrazin-8-one:

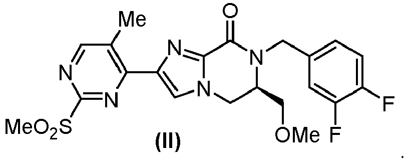

8. A compound of Formula **(III)**, (6*R*)-7-[(3,4-difluorophenyl)methyl]-6-(methoxymethyl)-2-(5-methyl-2-methylsulfanyl-pyrimidin-4-yl)-5,6-dihydroimidazo[1,2-a]pyrazin-8-one:

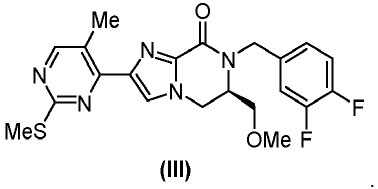

9. A compound of Formula **(IV),** (6R)-7-[(3,4-difluorophenyl)methyl]-6-(hydroxymethyl)-2-(5-methyl-2-methylsulfanyl-pyrimidin-4-yl)-5,6-dihydroimidazo[1,2-a]pyrazin-8-one:

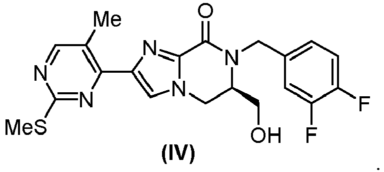

10. A compound of Formula **(V),** *N*-[(3,4-difluorophenyl)methyl]-4-(5-methyl-2-methylsulfanyl-pyrimidin-4-yl)-1-[[(2*R*)-oxiran-2-yl]methyl]imidazole-2-carboxamide:

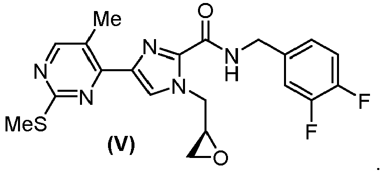

11. A compound of Formula **(VI),** *N*-[(3,4-difluorophenyl)methyl]-4-(5-methyl-2-methylsulfanyl-pyrimidin-4-yl)-1*H*-imidazole-2-carboxamide:

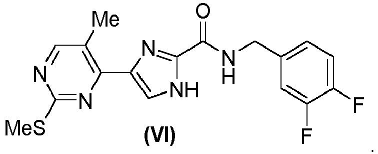

12. A compound of Formula **(VII),** 4-(5-methyl-2-methylsulfanyl-pyrimidin-4-yl)-1*H-*imidazole-2-carboxylic acid:

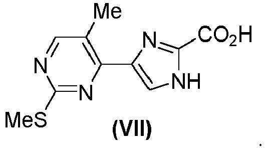

## Patentansprüche

1. Verfahren zur Herstellung einer Verbindung der Formel **(I):** 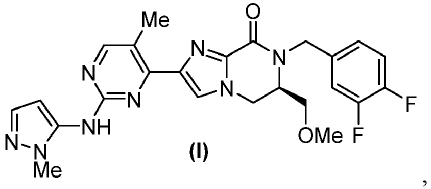 umfassend den Schritt der (i) Umsetzung einer Verbindung einer Verbindung der Formel **(II),** (6*R*)-7-[(3,4-Difluorophenyl)methyl]-6-(methoxymethyl)-2-(5-methyl-2-methylsulfonylpyrimidin-4-yl)-5,6-dihydroimidazo[1,2-a]pyrazin-8-on: 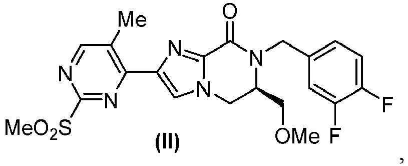 mit 2-Methylpyrazol-3-amin **(VIII):**

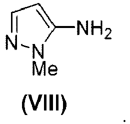

2. Verfahren nach Anspruch 1, umfassend den weiteren Schritt der (ii) Erzeugung der Verbindung der Formel **(II)** aus der Umsetzung einer Verbindung der Formel **(III),** (6*R*)-7-[(3,4-Difluorophenyl)methyl]-6-(methoxymethyl)-2-(5-methyl-2-methylsulfanyl-pyrimidin-4-yl)-5,6-dihydroimidazo[1,2-a]pyrazin-8-on: 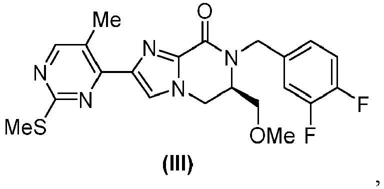 mit einem Oxidationsmittel.

3. Verfahren nach Anspruch 2, umfassend den weiteren Schritt der (iii) Erzeugung der Verbindung der Formel **(III)** aus der Umsetzung einer Verbindung der Formel **(IV),** (6*R*)-7-[(3,4-Difluorophenyl)methyl]-6-(hydroxymethyl)-2-(5-methyl-2-methylsulfanyl-pyrimidin-4-yl)-5,6-dihydroimidazo[1,2-a]pyrazin-8-on: 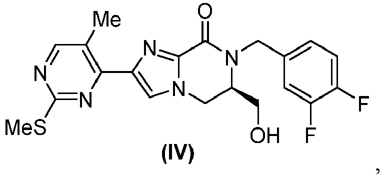 mit einem Methylierungsmittel.

4. Verfahren nach Anspruch 3, umfassend den weiteren Schritt der (iv) Erzeugung der Verbindung der Formel **(IV)** aus der Umsetzung einer Verbindung der Formel **(V),** *N*-[(3,4-Difluorophenyl)methyl]-4-(5-methyl-2-methylsulfanyl-pyrimidin-4-yl)-1-[[(2*R*)-oxiran-2-yl]methyl]imidazole-2-carboxamid: 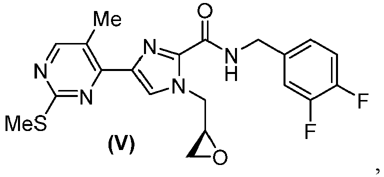 mit Lithiumbromid.

5. Verfahren nach Anspruch 4, umfassend den weiteren Schritt der (v) Erzeugung der Verbindung der Formel **(V)** aus der Umsetzung einer Verbindung der Formel **(VI),** *N*-[(3,4-Difluorophenyl)methyl]-4-(5-methyl-2-methylsulfanyl-pyrimidin-4-yl)-1*H*-imidazol-2-carboxamid: 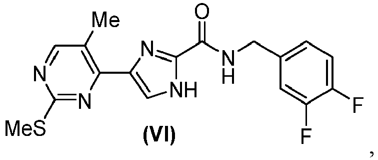 mit (*S*)-(+)-Epichlorohydrin **(IX):**

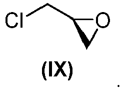

6. Verfahren nach Anspruch 5, umfassend den weiteren Schritt der (vi) Erzeugung der Verbindung der Formel **(VI)** aus der Umsetzung einer Verbindung der Formel **(VII),** 4-(5-Methyl-2-methylsulfanyl-pyrimidin-4-yl)-1*H*-imidazol-2-carbonsäure: 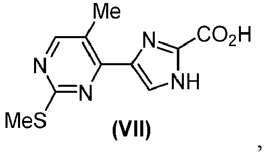 mit (3,4-Difluorophenyl)methanamin **(X):**

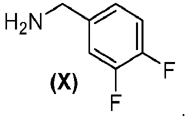

7. Verbindung der Formel **(II),** (6*R*)-7-[(3,4-Difluorophenyl)methyl]-6-(methoxymethyl)-2-(5-methyl-2-methylsulfonyl-pyrimidin-4-yl)-5,6-dihydroimidazo[1,2-a]pyrazin-8-on:

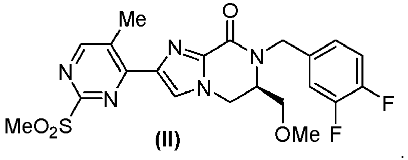

8. Verbindung der Formel **(III)**, (6*R*)-7-[(3,4-Difluorophenyl)methyl]-6-(methoxymethyl)-2-(5-methyl-2-methylsulfanyl-pyrimidin-4-yl)-5,6-dihydroimidazo[1,2-a]pyrazin-8-on:

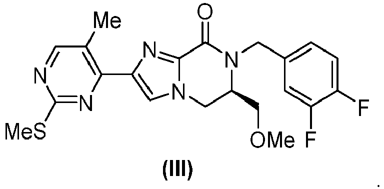

9. Verbindung der Formel **(IV),** (6*R*)-7-[(3,4-Difluorophenyl)methyl]-6-(hydroxymethyl)-2-(5-methyl-2-methylsulfanyl-pyrimidin-4-yl)-5,6-dihydroimidazo[1,2-a]pyrazin-8-on:

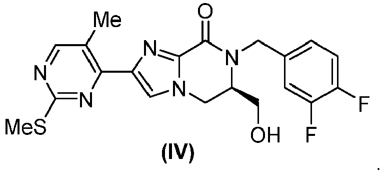

10. Verbindung der Formel **(V),** *N*-[(3,4-Difluorophenyl)methyl]-4-(5-methyl-2-methylsulfanyl-pyrimidin-4-yl)-1-[[(2*R*)-oxiran-2-yl]methyl]imidazol-2-carboxamid:

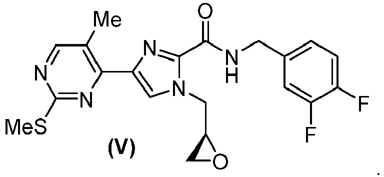

11. Verbindung der Formel **(VI),** *N*-[(3,4-Difluorophenyl)methyl]-4-(5-methyl-2-methylsulfanyl-pyrimidin-4-yl)-1*H*-imidazol-2-carboxamid:

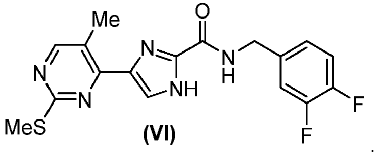

12. Verbindung der Formel **(VII),** 4-(5-methyl-2-methylsulfanyl-pyrimidin-4-yl)-1*H-*imidazol-2-carbonsäure:

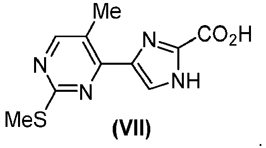

## Revendications

1. Procédé pour la production d'un composé de formule (I) : 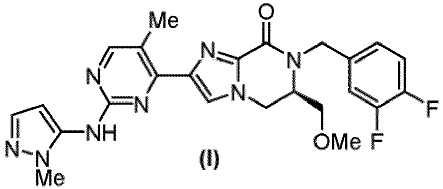 comprenant l'étape de (i) réaction d'un composé de formule (II), la (6*R*)-7-[(3,4-difluorophényl)méthyl]-6-(méthoxyméthyl)-2-(5-méthyl-2-méthylsulfonyl-pyrimidin-4-yl)-5,6-dihydroimidazo[1,2-a]pyrazin-8-one: 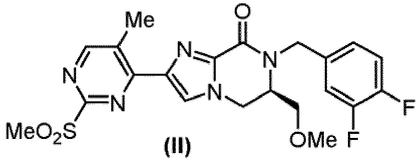 et de 2-méthylpyrazol-3 amine (VIII) :

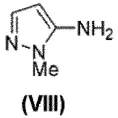

2. Procédé selon la revendication 1, comprenant l'étape supplémentaire de (ii) préparation du composé de formule (II) à partir de la réaction d'un composé de formule (III), la (6*R*)-7-[(3,4-difluorophényl)méthyl]-6-(méthoxyméthyl)-2-(5-méthyl-2-méthylsulfanylpyrimidin-4-yl)-5,6-dihydroimidazo[1,2-a]pyrazin-8-one : 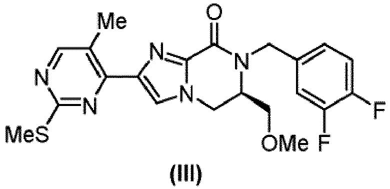 et d'un agent oxydant.

3. Procédé selon la revendication 2, comprenant l'étape supplémentaire de (iii) préparation du composé de formule (III) à partir de la réaction d'un composé de formule (IV), la (6*R*)-7-[(3,4-difluorophényl)méthyl]-6-(hydroxyméthyl)-2-(5-méthyl-2-méthylsulfanylpyrimidin-4-yl)-5,6-dihydroimidazo[1,2-a]pyrazin-8-one : 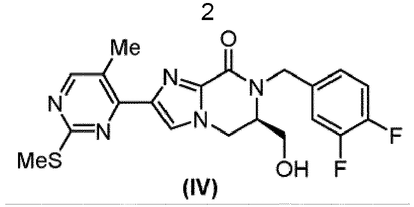 et d'un agent de méthylation.

4. Procédé selon la revendication 3, comprenant l'étape supplémentaire de (iv) préparation du composé de formule (IV) à partir de la réaction d'un composé de formule (V), le *N*-[(3,4-difluorophényl)méthyl]-4-(5-méthyl-2-méthylsulfanyl-pyrimidin-4-yl)-1-[[(2*R*)-oxiran-2-yl]méthyl]imidazole-2-carboxamide : 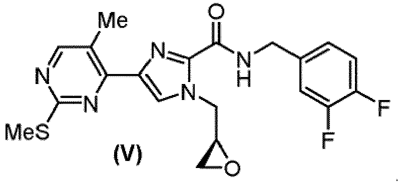 et de bromure de lithium.

5. Procédé selon la revendication 4, comprenant l'étape supplémentaire de (v) préparation du composé de formule (V) à partir de la réaction d'un composé de formule (VI), le *N*-[(3,4-difluorophényl)méthyl]-4-(5-méthyl-2-méthylsulfanyl-pyrimidin-4-yl)-1*H*-imidazole-2-carboxamide : 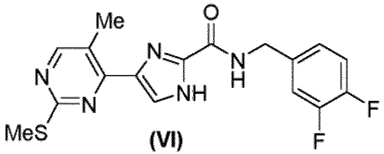 et de (S)-(+)-épichlorhydrine (IX) :

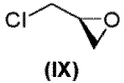

6. Procédé selon la revendication 5, comprenant l'étape supplémentaire de (vi) préparation du composé de formule (VI) à partir de la réaction d'un composé de formule (VII), l'acide 4-(5-méthyl-2-méthylsulfanyl-pyrimidin-4-yl)-1H-imidazole-2-carboxylique : 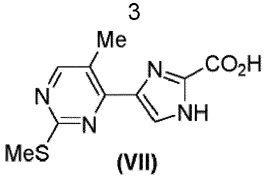 et de (3,4-difluorophényl)méthanamine (X) :

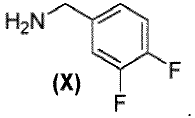

7. Composé de formule (II), la (6*R*)-7-[(3,4-difluorophényl)méthyl]-6-(méthoxyméthyl)-2-(5-méthyl-2-méthylsulfonyl-pyrimidin-4-yl)-5,6-dihydroimidazo[1,2-a]pyrazin-8-one :

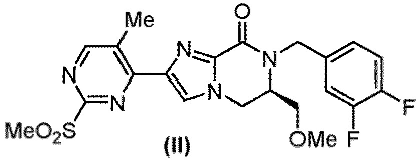

8. Composé de formule (III), la (6*R*)-7-[(3,4-difluorophényl)méthyl]-6-(méthoxyméthyl)-2-(5-méthyl-2-méthylsulfanyl-pyrimidin-4-yl)-5,6-dihydroimidazo[1,2-a]pyrazin-8-one :

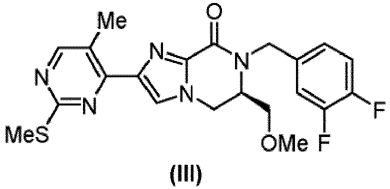

9. Composé de formule (IV), la (6*R*)-7-[(3,4-difluorophényl)méthyl]-6-(hydroxyméthyl)-2-(5-méthyl-2-méthylsulfanyl-pyrimidin-4-yl)-5,6-dihydroimidazo[1,2-a]pyrazin-8-one :

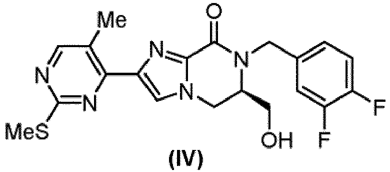

10. Composé de formule (V), le *N*-[(3,4-difluorophényl)méthyl]-4-(5-méthyl-2-méthylsulfanyl-pyrimidin-4-yl)-1-[[(2R)-oxiran-2-yl]méthyl]imidazole-2-carboxamide :

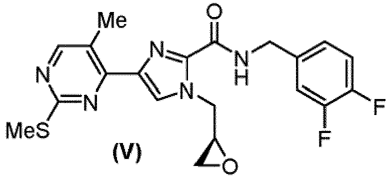

11. Composé de formule (VI), le *N*-[(3,4-difluorophényl)méthyl]-4-(5-méthyl-2-méthylsulfanyl-pyrimidin-4-yl)-1H imidazole-2-carboxamide :

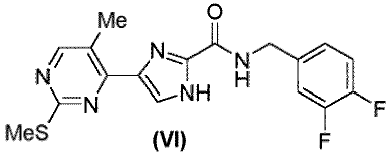

12. Composé de formule (VII), l'acide 4-(5-méthyl-2-méthylsulfanyl-pyrimidin-4-yl)-1H-imidazole-2-carboxylique :
